# EUROPÄISCHE PATENTSCHRIFT

(11) **EP 1 809 216 B1**
(45) Veröffentlichungstag und Bekanntmachung des Hinweises auf die Patenterteilung: **05.05.2010**
(21) Anmeldenummer: 05816540.8
(22) Anmeldetag: 08.11.2005
(51) Int. Cl.: A61F 5/01

(54) **Kippstabiles Drehgelenk und damit aufgebautes orthopädietechnisches Bauteil**
Tilt-stable rotating joint and technical orthopedic component constructed therewith
Articulation à charnière stable au basculement et composant orthopédique monté avec une telle articulation

(30) Priorität: 08.11.2004 DE 102004054384
(43) Veröffentlichungstag der Anmeldung: 25.07.2007
(73) Patentinhaber: Otto Bock HealthCare GmbH, 37115 Duderstadt (DE)
(72) Erfinder: WAGNER, Helmut, 37115 Duderstadt (DE)
(74) Vertreter: Lins, Edgar
(86) Internationale Anmeldenummer: PCT/DE2005/002025
(87) Internationale Veröffentlichungsnummer: WO 2006/048012

(56) Entgegenhaltungen:
- EP-A- 0 704 194
- GB-A- 228 284
- US-A- 5 370 701

## Beschreibung

Die Erfindung betrifft ein kippstabiles Drehgelenk mit einem gabelförmigen Außengelenkstück mit zwei Schenkeln, deren zueinander zeigende parallele Innenwandungen einen Schlitz vorgegebener Breite bilden und die mit miteinander fluchtenden Durchgangslöchern versehen sind, mit einem flachen, in den Schlitz ragenden Innengelenkstück mit einem Durchgangsloch und mit einer durch die Durchgangslöcher ragenden Achsanordnung, die mit einer Mantelfläche eine Drehlagerfläche bildet.

Die Erfindung betrifft ferner ein mit einem derartigen kippstabilen Drehgelenk aufgebautes orthopädietechnisches Bauteil.

Kippstabile Drehgelenke werden in vielfältigen Anwendungsfällen benötigt. Die Ausbildung eines kippstabilen Drehgelenks ist besonders problematisch, wenn für das Drehgelenk nur ein begrenzter Raum zur Verfügung steht und das Drehgelenk daher besonders flach ausgebildet sein muss, um nicht zu stark aufzutragen.

Drehgelenke dieser Art werden insbesondere in orthopädietechnischen Bauteilen benötigt, also in Orthesen oder Prothesen. Da die Drehgelenke dabei hohe sicherheitstechnische Anforderungen erfüllen müssen, müssen sie eine hohe Kippstabilität aufweisen und dürfen außerhalb ihrer Drehebene kein merkliches Spiel aufweisen.

Kippstabile Drehgelenke dieser Art, wie sie insbesondere für Orthesen benutzt werden, weisen ein flaches Innengelenkstück auf, in dessen Durchgangsloch ein Ring aus einem Gleitmetall eingepresst ist. Die Achsanordnung ist in Form einer Verschraubung so ausgebildet, dass die Schraubhülse mit ihrer glatten zylindrischen Mantelaußenfläche dem Durchmesser des eingepressten Rings aus Gleitmetall entspricht. Beide Teile der Verschraubung sind mit radial vorspringenden Köpfen versehen, die an der Außenseite der Schenkel, vorzugsweise versenkt, anliegen. Damit die Verschraubung drehfest mit den Schenkeln des gabelförmigen Außengelenkstücks verbunden ist, können einer der Köpfe und seine zugehörige Ausnehmung in dem Schenkel rotationsunsymmetrisch ausgebildet sein, sodass dieser Kopf in seiner Ausnehmung verdrehsicher gelagert ist und die Verschraubung daher nur auf der anderen Seite angezogen werden muss.

Die Kippstabilität des Drehgelenks ergibt sich aus einer präzisen Fertigung mit geringen Fertigungstoleranzen zwischen der zylindrischen Mantelaußenfläche der Verschraubung und dem eingepressten Ring aus einem Gleitmetall.

Durch EP 0 704 194 A1 ist ein Hüftgelenk für eine Orthese bekannt, bei der ein Drehgelenk um eine im Wesentlichen parallel zu der Frontalebene des Patienten ausgerichtete Drehachse dadurch realisiert, dass eine Gewindebuchse in eine Durchgangsöffnung der Schenkel eines gabelförmigen Außengelenkteils eingesetzt ist und dass eine Schraube mit einem Außengewindebolzen und einem größeren Schraubenkopf in die Gewindebuchse eingeschraubt ist. Die Mantelfläche der Gewindebuchse dient dabei als Gleitlager für ein zwischen die Schenkel des Außengelenkstücks eingesetztes Innengelenkstück, das eine dem Außendurchmesser der Gewindebuchse entsprechende Durchgangsöffnung aufweist. Auch dieses Gelenk weist die oben erwähnten Probleme hinsichtlich der Kippstabilität auf.

Durch US 5,370,701 ist eine implantierbare Kniegelenkprothese bekannt, bei der das Kniegelenk dem natürlichen Kniegelenk nachempfunden ist. Das zum Oberschenkel zeigende Anschlussteil des Kniegelenks ist daher mit einem den Kondylen des Oberschenkelknochens nachgebildeten Gelenkteil versehen, das sich auf einer gewölbten Tragplatte des Unterschenkelteils abwälzt. Das Oberschenkel-Gelenkteil weist dabei zwei im Bereich der Kondylen ausgebildete Schenkel auf, die mit miteinander fluchtenden Durchgangslöchern versehen sind. Fluchtend mit den Durchgangslöchern ist ein Durchgangsloch eines als Innengelenkteil fungierenden Joches des Unterschenkelteils angeordnet. Das Joch ragt dabei zwischen die Schenkel des Oberschenkel-Gelenkteils. Durch die Durchgangsöffnung ist ein Gelenkbolzen mit einem konstanten Außendurchmesser einschiebbar. In die Durchgangslöcher der beiden Schenkel des Oberschenkelteils sind Gleitbuchsen einsetzbar, sodass der Gelenkbolzen in den Gleitbuchsen der beiden Schenkel drehbar gelagert ist. Die beidseitige Lagerung ermöglicht eine verbesserte Kippstabilität, das bekannte Kniegelenk hat jedoch einen erheblichen Raumbedarf. Darüber hinaus ist ein gesondertes Feststellmittel in Form einer Feststellschraube erforderlich, um eine axiale Festlegung des Gelenkbolzens an dem Unterschenkelteil zu bewirken.

Der vorliegenden Erfindung liegt die Aufgabe zugrunde, ein Drehgelenk der eingangs erwähnten Art hinsichtlich seiner Kippstabilität zu verbessern und auf kleinem Raum aufbaubar zu machen.

Zur Lösung dieser Aufgabe ist ein kippstabiles Drehgelenk der eingangs erwähnten Art erfindungsgemäß **dadurch gekennzeichnet, dass** die durch das Durchgangsloch des Innengelenkstücks ragende Achsanordnung fest mit dem Innengelenkstück verbunden ist und aus dem Innengelenkstück herausragende Abschnitte mit einem gegenüber dem Durchgangsloch des Innengelenkstücks größeren Außendurchmesser aufweist, der dem Innendurchmesser der Durchgangslöcher der Schenkel entspricht, und dass zwischen den Wandungen der Durchgangslöcher der Schenkel und den Abschnitten der Achsanordnung Gleitlager ausgebildet sind.

Während bei einem herkömmlichen Drehgelenk der eingangs erwähnten Art die Drehlagerfläche mit der Durchgangsöffnung des Innengelenkstücks gebildet wird, sodass die Kippstabilität nur über eine ringförmige Lagerfläche erzeugt werden muss, die sich aus der Materialstärke des Innengelenkstücks ergibt, wird bei dem erfindungsgemäßen Drehgelenk eine drehfeste Einheit zwischen dem Innengelenkstück und der Achsanordnung ausgebildet, sodass die Abstützung des Gelenks zwischen der Achsanordnung und den Wandungen der Durchgangslöcher der Schenkel erfolgt. Die Abstützung wird somit an beiden zylindrischen Mantelflächen der Achsanordnung im Bereich der Schenkel bewirkt, sodass die Achsanordnung erfindungsgemäß an zwei voneinander beabstandeten Gleitlagern abgestützt wird. Dadurch werden eine erhebliche Vergrößerung der Gleitlagerfläche und eine verbesserte Kippstabilität erzielt. Da die aus dem Innengelenkstück herausragenden Abschnitte der Achsanordnung einen größeren Außendurchmesser aufweisen als das Durchgangsloch des Innengelenkstücks, ist gleichzeitig eine axiale Festlegung der Achsanordnung relativ zu dem Innengelenkstück sichergestellt, ohne dass hierfür zusätzliche Maßnahmen erforderlich wären.

In einer bevorzugten Ausführungsform der Erfindung sind auch ringförmige Ränder der Durchgangslöcher zwischen den Innenwandungen der Schenkel und den Oberflächen des Innengelenksstücks als Gleitlager ausgebildet. Vorzugsweise sind die Ränder der Innenwandungen als Gleitlager ausgebildet und stehen erhaben von der jeweiligen Innenwandung im Übrigen vor, sodass sich eine definierte ringförmige Anlagefläche zwischen Innenwandung und Innengelenkstück an Rand der Durchgangsöffnung ergibt.

Vorzugsweise wird die Wandung der Durchgangslöcher der Schenkel durch jeweils eine eingesetzte Gleitlagerhülse gebildet. Alternativ hierzu ist es möglich, auch die Oberfläche der Verschraubung in den Abschnitten des größten Außendurchmessers mit einer Gleitbeschichtung zu versehen.

In der bevorzugten Ausführungsform der in die Durchgangslöcher eingesetzten Gleitlagerhülsen sind diese jeweils mit einem kreisförmigen Flansch versehen, der an den Innenwandungen der Schenkel anliegt. Die eingesetzten Hülsen bilden somit sowohl die radialen Gleitlager in den Wandungen der Durchgangslöcher als auch die axialen ringförmigen Gleitlager an den Innenwandungen der Schenkel.

Die Materialstärke der Gleitlagerhülsen ist vorzugsweise kleiner als 1 mm und liegt bevorzugt zwischen 0,4 und 0,7 mm, vorzugsweise bei 0,5 mm.

Die Gleitlagerhülsen können in die Durchgangslöcher der Schenkel eingepresst sein. Andere Verbindungsarten, wie Kleben oder Schweißen sind ebenfalls möglich. Möglich ist ferner, die betreffenden Oberflächen durch eine chemische oder physikalische Oberflächenmodifikation als Gleitlager auszubilden.

In einer vorteilhaften Ausführungsform der Erfindung ist die Achsanordnung als Verschraubung mit einer Schraube und einer Schraubbuchse ausgebildet. Dabei können die aus den Innengelenkstücken herausragenden Abschnitte einen größeren Außendurchmesser aufweisen, sodass die Verschraubung im verschraubten Zustand eine radial offene Nut bildet, deren Durchmesser im Nutgrund dem Innendurchmesser des Durchgangslochs des Innengelenkstücks entspricht. Durch ein festes Anziehen der Verschraubung wird die drehfeste Verbindung zwischen der Verschraubung und dem Innengelenkstück bewirkt.

Die Nut der Verschraubung wird vorzugsweise durch einen Absatz des Durchmessers beider Teile der Verschraubung gebildet, obwohl es auch denkbar ist, nur ein Teil der Verschraubung mit einem Absatz auszubilden und so die Nut mit einem Nutgrund in nur einem Teil der Verschraubung bilden zu lassen.

Die Tiefe der radial offenen Nut kann sehr klein gehalten werden und beträgt beispielsweise 1 mm oder weniger. Der so gebildete Absatz reicht vollständig aus, um die Verbindung der Verschraubung mit dem Innengelenkstück zu einem praktisch einstückigen Gelenkteil zu bewirken.

Die Achsanordnung kann alternativ zu der Ausbildung als Verschraubung auch mit einer Nietanordnung ausgebildet sein, die mit Buchsen die Abschnitte für die Drehlagerfläche bildet.

Alternativ ist es möglich, in das Durchgangsloch des Innengelenkstücks einen Bolzen einzupressen, der mit seinen aus dem Innengelenkstück herausragenden Enden die Abschnitte für die Drehlagerfläche darstellt.

Selbstverständlich ist es in vielfältigen weiteren Varianten möglich, die drehfeste Verbindung zwischen der Achsanordnung und dem Innengelenkstück zu erreichen. Alle Verbindungsarten wie Verschränken, Anschweißen, Verkippen, Presspassen usw. sind denkbar, ebenso eine unsymmetrische Ausbildung des Durchgangslochs und eines durch das Durchgangsloch ragenden Bolzens der Achsanordnung, die außer des Innengelenkstücks natürlich die kreiszylindrischen Abschnitte aufweist, mit deren Mantelfläche die Gleitlagerfläche mit den Schenkeln des Außengelenkstücks ausgebildet wird.

Es ist selbstverständlich möglich, auf die oben erwähnte Verwendung von Gleitlagerhülsen zu verzichten, wenn für die Achsanordnung und die zylindrische Innenwandung der Durchgangslöcher in den Schenkeln des Außengelenkstücks ein entsprechend gleitfähiges Material verwendet wird, was durch eine entsprechende Materialauswahl und/oder eine Vergütung oder Beschichtung der entsprechenden Oberflächen erfolgen kann.

Das gabelförmige Außengelenkstück muss nicht einteilig ausgebildet sein, sondern kann aus zwei Teilen bestehen, die auch durch die Achsanordnung zusammengehalten werden können. Eine derartige Anordnung ist beispielsweise sinnvoll, wenn zwei Innengelenkstücke mit einer zahnförmigen Kontur ausgebildet sind und miteinander kämmen, sodass die Innengelenkstücke beide in einem Außengelenkstück gehalten werden, das durch zwei beiderseits der Innengelenkstücke angeordneten deckel artigen Teilen besteht, die die Schenkel des gabelförmigen Außengelenkstücks bilden.

Die Drehlagerflächen der Achsanordnung, die mit den entsprechenden Durchgangslöchern in den Schenkeln des Außengelenkstücks das Drehlager bilden, sind aus fertigungstechnischen Gründen vorzugsweise zylindrisch. Mit beispielsweise konischen Drehlagerflächen und Durchgangslöchern könnte allerdings - bei komplizierter Fertigung - eine noch höhere Kippstabilität erreicht werden.

Die Erfindung soll im Folgenden anhand von in der Zeichnung dargestellten Ausführungsbeispielen näher erläutert werden. Es zeigen:
- Figur 1: eine explodierte perspektivische Darstellung der Teile einer ersten Ausführungsform eines erfindungsgemäßen Drehgelenks
- Figur 2: eine explodierte Darstellung gemäß Figur 1 mit einem funktionsmäßig montierten Innengelenkstück
- Figur 3: eine Schnittdarstellung des montierten Drehgelenks
- Figur 4: eine Seitenansicht des montierten Drehgelenks
- Figur 5: eine Ansicht des montierten Drehgelenks von vorn
- Figur 6: eine andere Seitenansicht des montierten Drehgelenks
- Figur 7: eine perspektivische Ansicht des montierten Drehgelenks
- Figur 8: eine explodierte perspektivische Darstellung der Teile einer zweiten Ausführungsform eines erfindungsgemäßen Drehgelenks
- Figur 9: eine perspektivische Darstellung zur Verdeutlichung der aus Innengelenkstück und Achsanordnung gebildeten Einheit
- Figur 10: eine perspektivische Darstellung des montierten Drehgelenks gemäß der zweiten Ausführungsform
- Figur 11: eine explodierte perspektivische Darstellung der Teile einer dritten Ausführungsform eines erfindungsgemäßen Drehgelenks
- Figur 12: eine perspektivische Darstellung zur Verdeutlichung der durch die Achsanordnung und das Innengelenkstück gebildeten Einheit
- Figur 13: eine perspektivische Darstellung des montierten Drehgelenks gemäß der dritten Ausführungsform
- Figur 14: eine explodierte perspektivische Darstellung der Teile einer vierten Ausführungsform eines erfindungsgemäßen Drehgelenks
- Figur 15: eine perspektivische Darstellung zur Verdeutlichung der Ausbildung einer drehfesten Einheit aus Achsanordnung und Innengelenkstück
- Figur 16: eine perspektivische Darstellung des montierten Drehgelenks gemäß der vierten Ausführungsform
- Figur 17: eine explodierte perspektivische Darstellung der Teile einer fünften Ausführungsform eines erfindungsgemäßen Drehgelenks, das aus zwei miteinander kämmenden Innengelenkstücken und einem gemeinsamen Außengelenkstück besteht
- Figur 18: eine perspektivische Darstellung des montierten Drehgelenks gemäß der fünften Ausführungsform.

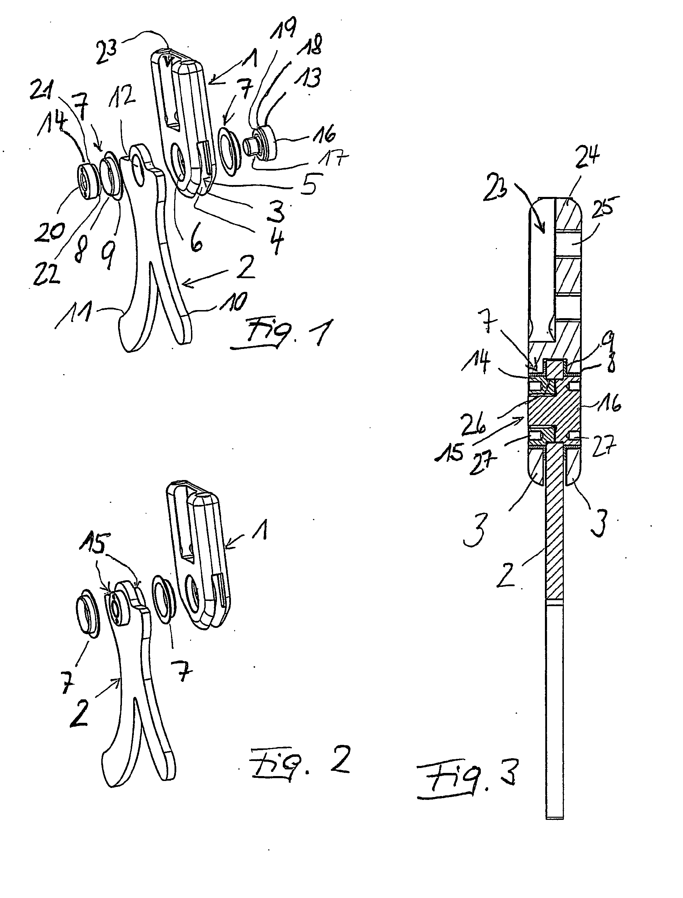

Das in den Figuren 1 bis 7 dargestellte erste Ausführungsbeispiel eines erfindungsgemäßen Drehgelenks weist ein Außengelenkstück 1 und ein Innengelenkstück 2 auf. Das Außengelenkstück 1 ist an einem Ende gabelförmig ausgebildet und weist zwei Schenkel 3 mit zueinander zeigenden parallelen Innenwandungen 4 auf, die einen Schlitz 5 einer vorgegebenen konstanten Breite bilden. Im Bereich des Schlitzes 5 sind die Schenkel 3 mit kreisrunden Durchgangslöchern 6 versehen, die auf beiden Schenkeln 3 gleich groß sind und miteinander fluchten. In die Durchgangslöcher 6 ist jeweils von der Schlitzseite eine Gleitlagerhülse 7 einsetzbar, die sich mit einem zum zylindrischen Buchsenteil 8 über die Breite des Schenkels 3, also über die gesamte Länge des Durchgangsloches 6, erstreckt. Ferner weist die Gleitlagerhülse 7 einen kreisringförmigen Flansch 9 auf, der somit an der Innenwandung 4 des zugehörigen Schenkels 3 anliegt.

Das Innengelenkstück 2 ist durch ein flaches Schienenstück gebildet, das eine für den jeweiligen Anwendungsfall geeignete Form aufweisen kann. In dem dargestellten Ausführungsbeispiel ist das Innengelenkstück 2 als unteres Knöchelgelenkstück mit zwei in entgegen gesetzten Richtungen abgebogenen Armen 10, 11 ausgebildet. Das Innengelenkstück 2 kann jede beliebige Form aufweisen, beispielsweise auch die Form einer rechteckigen Halteschiene. Das Innengelenkstück 2 besteht aus einem Flachmaterial, dessen Materialstärke an einem Ende so ausgebildet ist, dass das Innengelenkstück 2 mit diesem Ende in den Schlitz 5 des Außengelenkstücks 1 einsteckbar ist. An diesem Ende weist das Innengelenkstück 2 ein Durchgangsloch 12 auf, das im dargestellten Ausführungsbeispiel kreisförmig ausgebildet ist und einen um 1 mm kleineren Radius aufweist als die Durchgangslöcher 6 der Schenkel 3 des Außengelenkstücks 1.

Das Durchgangsloch 12 ist durch die Durchgangslöcher 6 zugänglich und wird durch eine Achsanordnung 15 relativ zum Außengelenkstück 1 fixiert, die eine aus einer Schraube 13 und einer Schraubbuchse 14 gebildete Verschraubung ist.

Die Schraube 13 besteht aus einem zylindrischen Schraubenkopf 16, der einstückig mit einem Gewindebolzen 17 versehen ist. Der Außendurchmesser des zylindrischen Schraubenkopfes entspricht passgenau dem Innendurchmesser des zylindrischen Buchsenteils 8 der Gleitlagerhülse 7, sodass die Gleitlagerhülse 7 mit dem Schraubenkopf 16 eine Drehlagerfläche bildet. Der Schraubenkopf 13 weist ferner einen kleinen, durch eine Durchmesserverringerung gebildeten Absatz 18 auf, durch den der Schraubenkopf 16 einen Abschnitt 19 aufweist, dessen Außendurchmesser dem Innendurchmesser des Durchgangslochs 12 des Innengelenkstücks 2 entspricht.

In analoger Weise bildet die Schraubbuchse 14 eine Durchgangsöffnung 20 mit einem Innengewinde zur Aufnahme des Gewindebolzens 17 und eine äußere zylindrische Mantelfläche, deren Außendurchmesser dem Innendurchmesser der Gleitlagerhülse 7 entspricht, auf. Die Breite der zylindrischen Mantelfläche entspricht der Breite des zylindrischen Buchsenteils 8 der Gleitlagerhülse. Im Anschluss daran ist die zylindrische Mantelfläche mit einem Absatz 21 versehen, an den sich ein zylindrischer Abschnitt 22 mit einem etwas verringerten Durchmesser anschließt. Der Außendurchmesser des zylindrischen Abschnitts 22 entspricht passgenau dem Innendurchmesser des Durchgangslochs 12.

Figur 2 verdeutlicht, dass die durch die Verschraubung gebildete Achsanordnung 15 mit dem Innengelenkstück 2 ein funktional einheitliches Teil bildet, das jedoch erst beim Zusammenbau des Drehgelenks zusammengestellt werden kann und nicht in der in Figur 2 dargestellten Weise vormontierbar ist.

Das Außengelenkstück 1 ist an seinem den Durchgangslöchern 6 gegenüberliegenden Ende mit einer rechteckigen Ausnehmung 23 versehen, die eine zur breiten Seite und zur Stirnseite offene Kammer bildet, in die das Endstück einer rechteckigen Flachschiene einsetzbar ist. Die Ausnehmung wird durch eine Rückwand 24 begrenzt, in der sich zwei Durchgangslöcher 25 zur Schraubbefestigung der in die Ausnehmung 23 eingesetzten Flachschiene befinden.

Figur 3 zeigt einen Hochschnitt durch das aus den in Figur 1 dargestellten Teilen montierte Drehgelenk. Es ist erkennbar, dass das Innengelenkstück 2 in einer Nut 26 der Verschraubung gehalten ist, die durch die Absätze 18, 21 und die daran anschließenden Abschnitte 19, 22 des Schraubenkopfs 16 und der Schraubbuchse 14 gebildet ist.

Schraubenkopf 16 und Schraubbuchse 14 weisen an ihren axialen Stirnflächen jeweils zwei diametral gegenüberliegende Sacklöcher 27 auf, die zum Drehen der Schraube 13 bzw. der Schraubbuchse 14 mittels eines geeigneten Werkzeugs dienen.

Das Innengelenkstück 2 wird passgenau in der Nut 26 der Verschraubung 15 radial gelagert und liegt axial an dem Flansch 9 der Gleitlagerhülse 7 an. Die Verschraubung liegt ihrerseits mit den zylindrischen Außenflächen von Schraubenkopf 16 und Schraubbuchse 14 an den zylindrischen Buchsenteilen 8 der Gleitlagerhülse 7 an, wodurch das eigentliche Drehlager für die Rotationsbewegung des Innengelenkstücks 2 relativ zum Außengelenkstück 1 gebildet wird. Bei der Drehbewegung wird regelmäßig eine Relativbewegung des Innengelenkstücks 2 zur Verschraubung nicht stattfinden. Die Verbindung zwischen Innengelenkstück 2 und Verschraubung kann durch einen Formschluss drehfest ausgestaltet sein, der z. B. durch ein Vierkantloch im Innengelenkstück 2 und einer entsprechenden Ausbildung der Absätze 19, 21 der Verschraubung gebildet sein kann. In diesem Fall muss die Verschraubung durch eine zusätzliche Schraube komplettiert werden.

Während bei herkömmlichen Drehlagern dieser Art das Drehlager an der Innenwand des Durchgangslochs 12 des Innengelenkstücks 2 gegenüber einer zylindrischen Verschraubung gebildet ist und sich daher nur über die Materialstärke des Innengelenstücks 2 gegen Kippbewegungen abstützen kann, findet bei der dargestellten ersten Ausführungsform des erfindungsgemäßen Drehgelenks die Abstützung für die Rotationsbewegung an den beiden Buchsenteilen 8 der Gleitlagerhülsen 7 statt und erstreckt sich somit über die Länge der Verschraubung bzw. die Gesamtdicke des Außengelenkstücks mit den beiden Schenkeln 3, sodass eine deutlich erhöhte Kippstabilität gewährleistet ist.

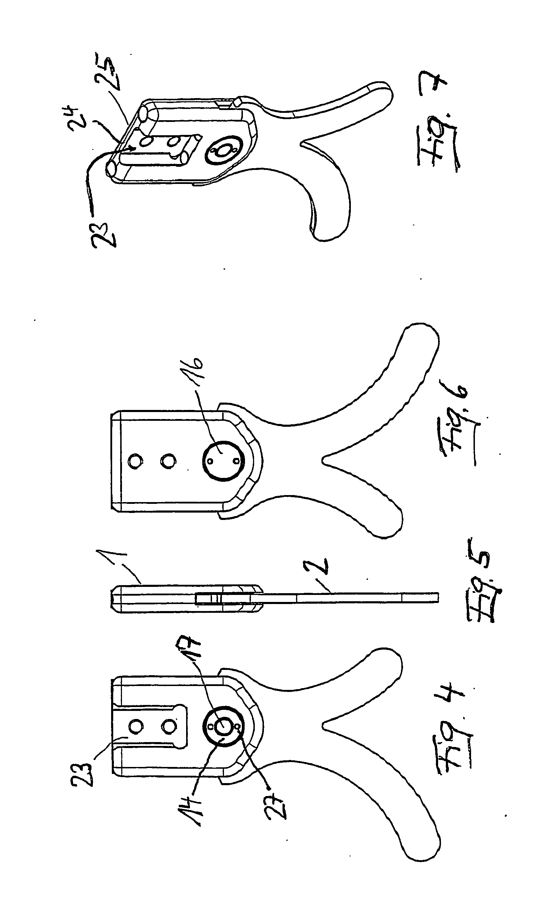

Figur 4 zeigt eine Seitenansicht des montierten Drehgelenks auf die Seite, auf der sich die Ausnehmung 23 und die Schraubbuchse 14 befinden. Erkennbar ist in der Schraubbuchse 14 das freie Ende des Gewindebolzens 17.

Figur 5 zeigt eine Ansicht des montierten Drehgelenks von vorn und lässt erkennen, dass das Drehgelenk mit einem sehr schmalen Aufbau erstellt werden kann, der, beispielsweise bei der Verwendung als Orthesengelenk, an gestützten Gliedmaßen eines menschlichen Körpers nicht wesentlich aufträgt.

Figur 6 zeigt eine Seitenansicht auf die Seite des montierten Drehgelenks, auf der sich der Schraubenkopf 16 befindet.

Figur 7 verdeutlicht das montierte Drehgelenk gemäß der ersten Ausführungsform in einer perspektivischen Darstellung.

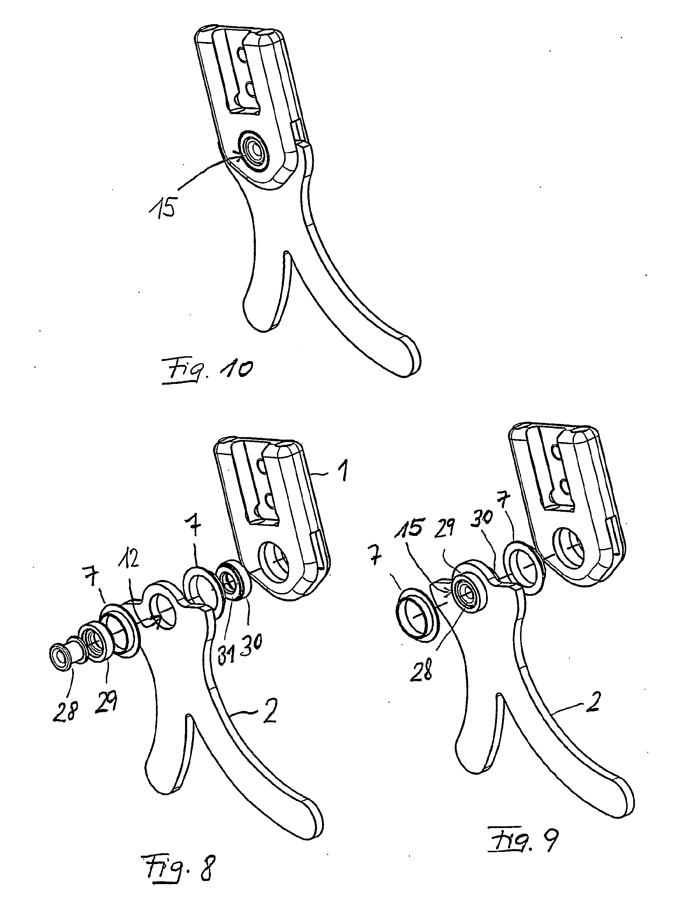

In den Figuren 8 bis 10 ist eine zweite Ausführungsform eines erfindungsgemäßen Drehgelenks dargestellt. Dieses unterscheidet sich von der ersten Ausführungsform lediglich in einer anderen Ausbildung der Achsanordnung 15, die in dieser Ausführungsform durch einen Niet 28 und zwei Buchsen 29, 30 gebildet ist, die mit Absätzen 31 mit etwas verringertem Außendurchmesser in das Durchgangsloch des Innengelenkstücks 2 ragen, sodass sie Abschnitte mit einem größeren Außendurchmesser bilden, die zusammen mit den Gleitlagerhülsen 7 das Gleitlager zwischen dem Außengelenkstück 1 und dem Innengelenkstück 2 bilden.

Figur 9 zeigt, dass die beiden Buchsen 29, 30 durch den Niet 28 zusammengehalten und gegen das Material des Innengelenkstücks 2 gedrückt werden, sodass eine drehfeste Verbindung zwischen den Buchsen 29, 30 und dem Innengelenkstück 2 besteht. Die Darstellung in Figur 9 ist unrealistisch, weil die Befestigung der Buchsen an dem Innengelenkstück 2 mittels des Niets 28 erst erfolgen kann, wenn das Innengelenkstück 2 in das Außengelenkstück 1 passend eingesetzt ist. Die Darstellung der Figur 9 dient daher lediglich zur Erläuterung der Befestigung der Buchse 29, 30 an dem Innengelenkstück 2 im montierten Zustand, wie er sich in Figur 10 darstellt.

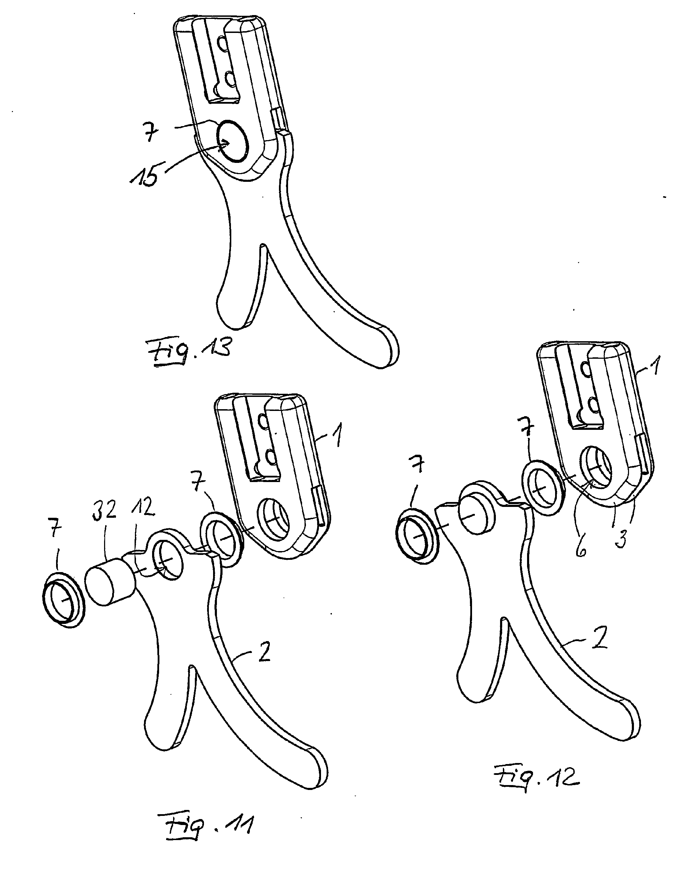

Bei der in den Figuren 11 bis 13 dargestellten dritten Ausführungsform eines erfindungsgemäßen Drehgelenks ist die Achsanordnung 15 durch einen Bolzen 32 gebildet, der so dimensioniert ist, dass er in der Durchgangsöffnung 12 des Innengelenkstücks 2 mittels Presssitz gehalten wird, wobei, wie Figur 12 verdeutlicht, beidseitig des Innengelenkstücks 2 Abschnitte des Bolzens herausragen, die mit den Gleitlagerhülsen 7, die in die Durchgangslöcher 6 in den Schenkeln 3 des Außengelenkstücks 1 eingesetzt werden, das in Figur 13 in montiertem Zustand gezeigte Gleitlager bilden.

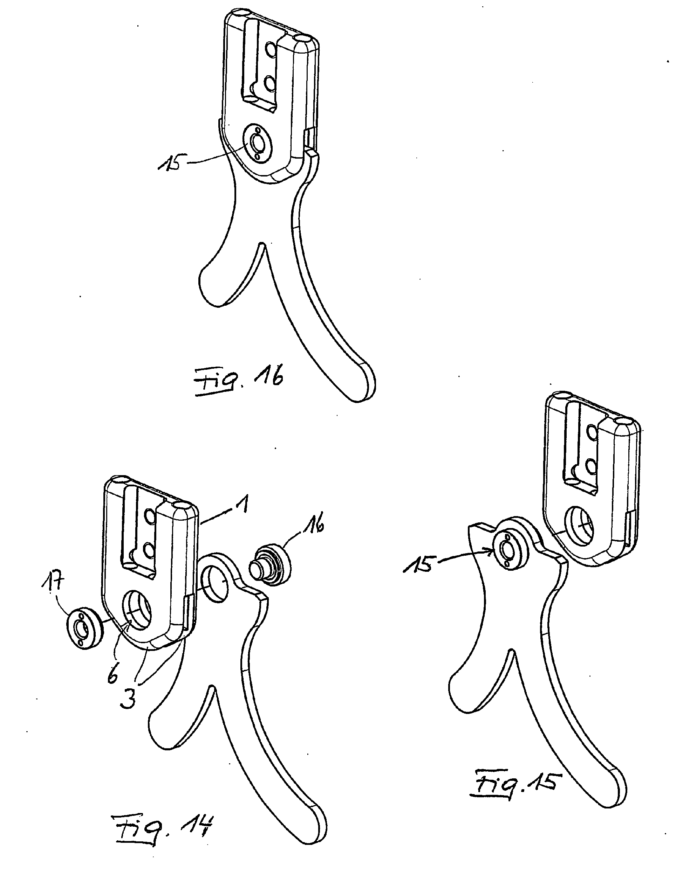

Die in den Figuren 14 bis 16 dargestellte vierte Ausführungsform der Erfindung entspricht der ersten Ausführungsform der Figuren 1 bis 7, wobei jedoch auf den Einsatz von Gleithülsen 7 verzichtet worden ist, weil der Schraubenkopf 16 und der Gewindebolzen 17 und die Innenwandung der Durchgangslöcher 6 in den Schenkeln 3 des Außengelenkstücks 1 als Gleitlager ausgebildet sind, was durch eine entsprechende Materialauswahl oder eine Oberflächenbehandlung der Gleitlagerflächen möglich ist.

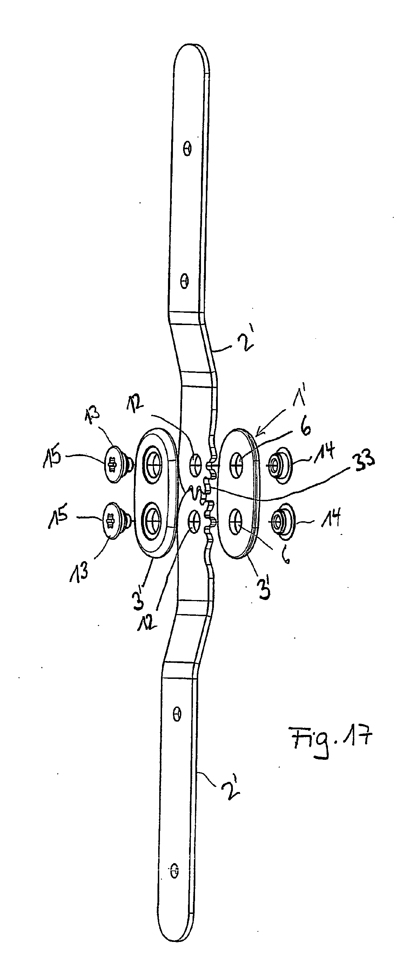

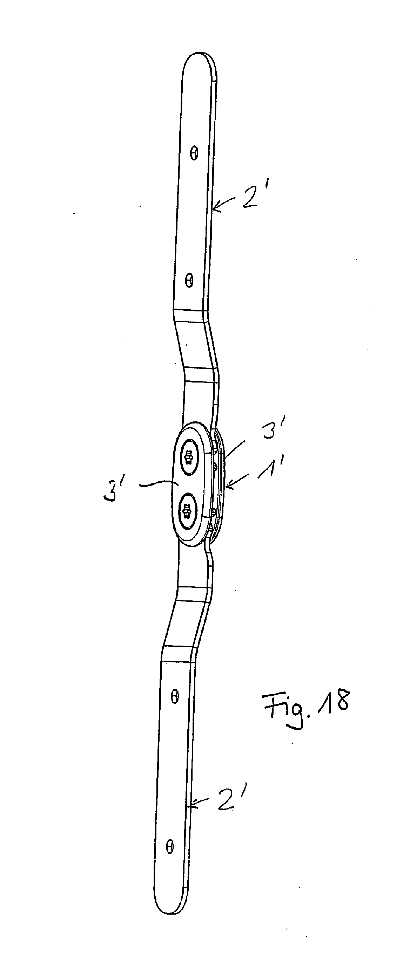

Die in den Figuren 17 und 18 dargestellte fünfte Ausführungsform eines erfindungsgemäßen Drehgelenks besteht aus zwei Innengelenkstücken 2' und einem Außengelenkstück 1', das aus zwei die Schenkel 3' bildenden Einzelteilen besteht. Das Außengelenkstück 1' ist somit beiden Innengelenkstücken 2' gemeinsam. Demgemäß weisen die Schenkel 3' des Außengelenkstücks 1' zwei Durchgangslöcher 6 auf, die mit jeweils einem Durchgangsloch 12 eines der Innengelenkstücke 2' fluchten.

Demgemäß wird das Drehgelenk durch zwei Achsanordnungen 15 gebildet, die, wie in der beschriebenen vierten Ausführungsform, durch eine aus Schraube 13 und Schraubbuchse 14 gebildete Verschraubung bestehen. Auch für diese Ausführungsform kann aufgrund einer geeigneten Materialwahl oder -ausbildung auf die Verwendung von Gleitlagerhülsen 7 verzichtet werden.

Die beiden Innengelenkstücke 2' stoßen zwischen den Achsanordnungen 15 mit jeweils einer Außenverzahnung 33 aufeinander, die miteinander kämmen. Dadurch werden die Drehbewegungen der beiden Innengelenkstücke 2' relativ zum Außengelenkstück 1' synchronisiert, was für die Ausbildung eines Kniegelenks, beispielsweise für eine Orthese, sinnvoll ist. Hierfür ist die Form der Innengelenkstücke 2' angepasst.

Das erfindungsgemäße Drehgelenk ermöglicht ohne zusätzlichen Aufwand eine erhöhte Kippstabilität bei einem schmalen Aufbau des Drehgelenks.

## Patentansprüche

1. Kippstabiles Drehgelenk mit einem gabelförmigen Außengelenkstück (1) mit zwei Schenkeln (3), deren zueinander zeigende parallele Innenwandungen (4) einen Schlitz (5) vorgegebener Breite bilden und die mit miteinander fluchtenden Durchgangslöchern (6) versehen sind, mit einem flachen, in den Schlitz (5) ragenden Innengelenkstück (2) mit einem Durchgangsloch (12) und mit einer durch die Durchgangslöcher (6, 12) ragenden Achsanordnung (15), die mit einer Mantelfläche eine Drehlagerfläche bildet, **dadurch gekennzeichnet, dass** die durch das Durchgangsloch (12) des Innengelenkstücks (2) ragende Achsanordnung (15) drehfest mit dem Innengelenkstück (2) verbunden ist und aus dem Innengelenkstück (2) herausragende Abschnitte mit einem gegenüber dem Durchgangsloch (12) des Innengelenkstücks (2) größeren Außendurchmesser aufweist, der dem Innendurchmesser der Durchgangslöcher (6) der Schenkel (3) entspricht, und dass zwischen den Wandungen der Durchgangslöcher (6) der Schenkel (3) und den Abschnitten der Achsanordnung (15) Gleitlager ausgebildet sind.

2. Drehgelenk nach Anspruch 1, **dadurch gekennzeichnet, dass** auch ringförmige Ränder der Durchgangslöcher (6) zwischen den Innenwandungen (4) der Schenkel (3) und den Oberflächen des Innengelenkstücks (2) als Gleitlager ausgebildet sind.

3. Drehgelenk nach Anspruch 2, **dadurch gekennzeichnet, dass** die ringförmigen Ränder erhaben von der jeweiligen Innenwandung (4) im Übrigen vorsteht.

4. Drehgelenk nach einem der Ansprüche 1 bis 3, **dadurch gekennzeichnet, dass** die Wandung der Durchgangslöcher (6) der Schenkel (3) durch jeweils eine eingesetzte Gleitlagerhülse (7) gebildet ist.

5. Drehgelenk nach Anspruch 4, **dadurch gekennzeichnet, dass** die Gleitlagerhülsen (7) jeweils mit einem kreisförmigen Flansch (9) versehen sind, der an der Innenwandung (4) des zugehörigen Schenkels (3) anliegt.

6. Drehgelenk nach Anspruch 4 oder 5, **dadurch gekennzeichnet, dass** die Materialstärke der Gleitlagerhülsen (7) kleiner als 1 mm ist.

7. Drehgelenk nach Anspruch 6, **dadurch gekennzeichnet, dass** die Materialstärke zwischen 0,4 und 0,7 mm liegt.

8. Drehgelenk nach einem der Ansprüche 4 bis 7, **dadurch gekennzeichnet, dass** die Gleitlagerhülsen (7) in die Durchgangslöcher (6) der Schenkel (3) eingepresst sind.

9. Drehgelenk nach einem der Ansprüche 1 bis 8, **dadurch gekennzeichnet, dass** die Achsanordnung (15) durch eine Verschraubung aus einer Schraube (13) und einer Schraubbuchse gebildet ist.

10. Drehgelenk nach Anspruch 9, **dadurch gekennzeichnet, dass** die Verschraubung im verschraubten Zustand eine radial offene Nut (26) bildet, deren Durchmesser im Nutgrund dem Innendurchmesser des Durchgangslochs (12) des Innengelenkstücks (2) entspricht.

11. Drehgelenk nach Anspruch 9 oder 10, **dadurch gekennzeichnet, dass** die Verschraubung (15) im Bereich der größten Außendurchmesser mit einem Gleitlagerwerkstoff versehen ist.

12. Drehgelenk nach einem der Ansprüche 1 bis 11, **dadurch gekennzeichnet, dass** die Nut (26) der Verschraubung (15) durch einen Absatz (21) des Durchmessers wenigstens eines Teils (13, 14) der Verschraubung (15) gebildet ist.

13. Drehgelenk nach Anspruch 12, **dadurch gekennzeichnet, dass** sich der Absatz (21) sowohl auf der Schraubbuchse (14) als auch auf der Schraube (13) befindet.

14. Orthopädietechnisches Bauteil mit wenigstens einem kippstabilen Drehgelenk nach einem der Ansprüche 1 bis 13.

## Claims

1. Tilt-stable rotating joint with a fork-shaped outer joint piece (1) having two branches (3) whose parallel inner walls (4) facing each other form a slit (5) of predetermined width and are provided with mutually aligned through-holes (6), with a flat inner joint piece (2) which protrudes into the slit (5) and has a through-hole (12), and with a shaft arrangement (15) which protrudes through the through-holes (6, 12) and, together with a jacket surface, forms a rotary bearing surface, **characterized in that** the shaft arrangement (15) protruding through the through-hole (12) of the inner joint piece (2) is connected in a rotationally fixed manner to the inner joint piece (2) and has sections that protrude from the inner joint piece (2) and that have an external diameter which is larger than the through-hole (12) of the inner joint piece (2) and which corresponds to the internal diameter of the through-holes (6) of the branches (3), and **in that** slide bearings are formed between the walls of the through-holes (6) of the branches (3) and the sections of the shaft arrangement (15).

2. Rotating joint according to Claim 1, **characterized in that** annular edges of the through-holes (6) between the inner walls (4) of the branches (3) and the surfaces of the inner joint piece (2) are also designed as slide bearings.

3. Rotating joint according to Claim 2, **characterized in that** the annular edges protrude as raised edges from the rest of the respective inner wall (4).

4. Rotating joint according to one of Claims 1 through 3, **characterized in that** the wall of the through-holes (6) of the branches (3) is formed by in each case an inserted slide-bearing sleeve (7).

5. Rotating joint according to Claim 4, **characterized in that** the slide-bearing sleeves (7) are each provided with a circular flange (9) that bears on the inner wall (4) of the associated branch (3).

6. Rotating joint according to Claim 4 or 5,
**characterized in that** the material thickness of the slide-bearing sleeves (7) is less than 1 mm.

7. Rotating joint according to Claim 6, **characterized in that** the material thickness is between 0.4 and 0.7 mm.

8. Rotating joint according to one of Claims 4 through 7, **characterized in that** the slide-bearing sleeves (7) are pressed into the through-holes (6) of the branches (3).

9. Rotating joint according to one of Claims 1 through 8, **characterized in that** the shaft arrangement (15) is formed by a screwed union composed of a screw (13) and of a screw bushing.

10. Rotating joint according to Claim 9, **characterized in that** the screwed union, when screwed together, forms a radially open groove (26) whose diameter, at the groove base, corresponds to the internal diameter of the through-hole (12) of the inner joint piece (2).

11. Rotating joint according to Claim 9 or 10,
**characterized in that** the screwed union (15) is provided with a slide-bearing material in the area of the greatest external diameter.

12. Rotating joint according to one of Claims 1 through 11, **characterized in that** the groove (26) of the screwed union (15) is formed by a shoulder (21) in the diameter of at least one part (13, 14) of the screwed union (15).

13. Rotating joint according to Claim 12,
**characterized in that** the shoulder (21) is located both on the screw bushing (14) and also on the screw (13).

14. Technical orthopedic component having at least one tilt-stable rotating joint according to one of Claims 1 through 13.

## Revendications

1. Articulation à charnière stable à l'égard du basculement comprenant ; une pièce d'articulation externe (1) en forme de fourche, ayant deux branches (3), dont les parois internes (4) parallèles se faisant face mutuellement forment une fente (5) de largeur prédéterminée, et qui sont munies de trous traversants (6) alignés ensemble ; une pièce d'articulation interne (2) plate s'engageant dans la fente (5), ayant un trou traversant (12) ; et, s'engageant à travers les trous traversants (6, 12), un agencement d'axe (15) dont une surface latérale forme une surface de pivotement, **caractérisée en ce que** l'agencement d'axe (15) s'engageant à travers le trou traversant (12) de la pièce d'articulation interne (2) est solidaire en rotation de la pièce d'articulation interne (2) et présente des tronçons faisant saillie hors de la pièce d'articulation interne (2), ayant un diamètre extérieur plus grand comparativement au trou traversant (12) de la pièce d'articulation interne (2) et qui correspond au diamètre interne des trous traversants (6) des branches (3), et **en ce que** des paliers lisses sont formés entre les parois des trous traversants (6) des branches (3) et les tronçons de l'agencement d'axe (15).

2. Articulation selon la revendication 1, **caractérisée en ce que** des bordures annulaires des trous traversants (6) sont aussi formées en tant que paliers lisses, entre les parois internes (4) des branches (3) et les surfaces de la pièce d'articulation interne (2).

3. Articulation selon la revendication 2, **caractérisée en ce que** les bordures annulaires dépassent du reste de la paroi interne (4) respective.

4. Articulation selon l'une des revendications 1 à 3, **caractérisée en ce que** la paroi des trous traversants (6) des branches (3) est formée par un manchon de palier lisse (7) respectif qui est introduit.

5. Articulation selon la revendication 4, **caractérisée en ce que** les manchons de palier lisse (7) sont munis chacun d'une collerette (9) circulaire qui est appuyée sur la paroi interne (4) de la branche (3) correspondante.

6. Articulation selon la revendication 4 ou 5, **caractérisée en ce que** l'épaisseur de matériau des manchons de palier lisse (7) est inférieure à 1 mm.

7. Articulation selon la revendication 6, **caractérisée en ce que** l'épaisseur de matériau vaut entre 0,4 et 0,7 mm.

8. Articulation selon l'une des revendications 4 à 7, **caractérisée en ce que** les manchons de palier lisse (7) sont montés à force dans les trous traversants (6) des branches (3).

9. Articulation selon l'une des revendications 1 à 8, **caractérisée en ce que** l'agencement d'axe (15) est formé par un assemblage vissé à base d'une vis (13) et d'une douille filetée.

10. Articulation selon la revendication 9, **caractérisée en ce que** l'assemblage vissé forme à l'état vissé une rainure (26) ouverte radialement, dont le diamètre dans le fond de la rainure correspond au diamètre interne du trou traversant (12) de la pièce d'articulation interne (2).

11. Articulation selon la revendication 9 ou 10, **caractérisée en ce que** l'assemblage vissé (15) est muni d'un matériau de palier lisse dans la région de plus grand diamètre externe.

12. Articulation selon l'une des revendications 1 à 11, **caractérisée en ce que** la rainure (26) de l'assemblage vissé (15) est formée par un retrait (21) du diamètre d'au moins une partie (13, 14) de l'assemblage vissé (15).

13. Articulation selon la revendication 12, **caractérisée en ce que** le retrait (21) se trouve aussi bien sur la douille filetée (14) que sur la vis (13).

14. Composant orthopédique comprenant au moins une articulation à charnière stable à l'égard du basculement selon l'une des revendications là 13.
